# EUROPÄISCHE PATENTANMELDUNG

(11) **EP 1 839 684 A1**
(43) Veröffentlichungstag der Anmeldung: **03.10.2007**
(21) Anmeldenummer: 06006372.4
(22) Anmeldetag: 28.03.2006
(51) Int. Cl.: A61L 9/04, A61L 9/12, A45D 34/02, B60H 3/00, B65D 83/00, E03D 9/00

(54) **Duftspender**

(71) Anmelder: OPAN AG, 8834 Schindellegi (CH)
(72) Erfinder: Budich, Meinrad, 32609 Hüllhorst (DE)
(74) Vertreter: Beckord, Klaus

(57) **Zusammenfassung**

Die Erfindung betrifft einen Duftspender (1) zur Abgabe eines flüssigen, flüchtigen Duftstoffes (3) in die Umgebungsluft. Der Duftspender (1) weist eine den flüssigen Duftstoff (3) aufnehmenden Flasche (2) mit einer durch eine Kappe (4) verschließbaren Öffnung (7) und einen von der Öffnung (7) aus in die Flasche (2) hineinragenden Dochthalter (5) mit einem daran befestigten Docht (6) auf, welcher im Gebrauch des Duftspenders (1) teilweise in den flüssigen Duftstoff (3) eintaucht und teilweise aus dem Duftstoff (3) herausragt. Dabei ist der Docht (6) mittels eines Clip-Elements (20) am Dochthalter (5) fixiert. Darüber hinaus betrifft die Erfindung ein Verfahren zur Herstellung eines solchen Duftspenders (1).

## Beschreibung

Die Erfindung betrifft einen Duftspender zur Abgabe eines flüssigen, flüchtigen Duftstoffes in die Umgebung mit einer den flüssigen Duftstoff aufnehmenden Flasche mit einer durch eine Kappe verschließbaren Öffnung. Der Duftspender besitzt einen an einem Dochthalter befestigten Docht, der von der Öffnung aus in die Flasche hineinragt und der beim Gebrauch des Duftspenders teilweise in den flüssigen Duftstoff eintaucht und teilweise aus dem Duftstoff herausragt. Darüber hinaus betrifft die Erfindung einen entsprechenden Dochthalter für einen solchen Duftspender sowie ein Verfahren zur Herstellung eines derartigen Duftspenders.

Duftspender der eingangs genannten Art sind in ganz unterschiedlichen Ausgestaltungen aus dem Stand der Technik bekannt. Sie dienen dazu, die unmittelbare Umgebung, beispielsweise die Atmosphäre in einem Raum, zu parfümieren und/oder zu desodorieren. Die Funktionsweise dieser Duftspender ist derart, dass der flüssige Duftstoff in den Docht eindringt, in diesem nach oben steigt und dann vom Docht abdampft. Die Abdampfrate (d. h. wie stark der Duftspender den flüchtigen Duftstoff in die Umgebung abgibt) lässt sich auf verschiedene Weise einstellen. Bei einigen Vorrichtungen geschieht dies dadurch, dass die Kappe mehr oder weniger weit von der Flasche abgeschraubt wird. Bei anderen Duftspendern wird die Abdampfrate dadurch eingestellt, dass der Docht mehr oder weniger hoch aus der Flasche herausgezogen wird. Typische Beispiele eines solchen Duftspenders werden in der GB 738,173 und der FR 2 683 149 beschrieben. Die Dochthalter sind hierbei jeweils so ausgestaltet, dass sie in verschiedenen Höhen innerhalb des Flaschenhalses festgestellt werden können und somit der Anteil des Dochts, der durch den Flaschenhals aus der Flasche herausragt und der somit in unmittelbarem Kontakt mit der Umgebungsluft ist, eingestellt werden kann. Die Duftspender der eingangs genannten Art haben neben der einfachen Einstellbarkeit der Abdampfrate den Vorteil, dass sie relativ großvolumig sind und daher eine lange Betriebszeit aufweisen, bis sie wieder befüllt bzw. üblicherweise komplett ausgetauscht werden müssen. Ein weiterer Vorteil besteht darin, dass sie relativ flexibel mit verschiedensten Duftstoffen ausgerüstet werden können. Das heißt, wenn ein neuer Duftstoff kreiert wird, ist es nicht notwendig, die Konstruktion des Duftspenders zu verändern, sondern es brauchen nur die Duftspenderflaschen mit dem neuen flüssigen Duftstoff befüllt und entsprechend etikettiert zu werden. Ungünstig bei diesen Duftspendern ist jedoch bisher noch die Halterung des Dochts in der Flasche. Üblicherweise werden hierzu längliche, lamellenförmige Dochthalter aus Kunststoff verwendet, um die als Docht ein langer Streifen aus Fließmaterial herumgewickelt wird. Dieser Dochtstreifen wird dann, wie dies in der FR 2 683 149 beschrieben ist, durch Aussparungen im Dochtträger in einer bestimmten Weise kunstvoll hindurchgefädelt, um den Docht sicher am Dochthalter zu fixieren. Diese umständliche Befestigung des Dochts am Dochthalter ist bisher nur in relativ zeitaufwändiger Handarbeit möglich. Hinzu kommt, dass der Fließstreifen relativ dünn sein muss, um den Docht in die Aussparungen des Dochthalters einfädeln zu können. Andererseits sollte der gesamte Docht aber eine bestimmte Dicke aufweisen, um in der Gebrauchsstellung den Flaschenhals möglichst dicht abzuschließen, so dass zum einen die Abdampfrate allein durch den aus dem Flaschenhals nach oben herausragenden Dochtabschnitt bestimmt und zum anderen vermieden wird, dass der flüssige Duftstoff sofort ausläuft, wenn die Flasche im geöffneten Zustand umgestoßen wird. Daher ist es im Allgemeinen erforderlich, den Dochtstreifen mehrfach um den Dochthalter zu schlingen, um die notwendige Stärke zu erreichen. Dies erhöht den Zeitaufwand zur Fixierung des Dochts am Dochthalter ebenfalls.

Es ist daher eine Aufgabe der vorliegenden Erfindung, einen Duftspender der eingangs genannten Art, einen hierfür geeigneten Dochthalter sowie ein Verfahren zur Herstellung eines solchen Duftspenders zu schaffen, wobei die Fixierung des Dochts am Dochthalter erheblich schneller und kostengünstiger durchführbar ist.

Diese Aufgabe wird zum einen durch einen Duftspender nach Patentanspruch 1 sowie einen Dochthalter nach Patentanspruch 16 und zum anderen durch ein Verfahren gemäß Patentanspruch 17 gelöst.

Kernidee der Erfindung ist dabei, den Docht nicht mehr wie bisher umständlich in eine Ausnehmung des Dochthalters einzufädeln, sondern stattdessen den Docht einfach mittels eines geeigneten Clip-Elements am Dochthalter zu fixieren. Dadurch kann die Befestigung - auch wenn sie manuell durchgeführt wird - erheblich schneller als bei den bisher bekannten Duftspendern erfolgen. Darüber hinaus ist bei entsprechender Ausgestaltung des Clip-Elements und des Dochts auch ohne weiteres eine ggf. noch kostengünstigere, automatische Bestückung des Dochthalters möglich. Das Verfahren hat zudem den Vorteil, dass ein erheblich dickeres Dochtmaterial als bisher eingesetzt werden kann und daher ein mehrfaches Herumwickeln des Dochts nicht mehr notwendig ist.

Ein erfindungsgemäßer Dochthalter für einen Duftspender muss daher zunächst in üblicher Weise einerseits ein Halterungselement aufweisen, um den Dochthalter in einer den flüssigen Duftstoff aufnehmenden Flasche des Duftspenders so zu halten, dass er von einer Öffnung der Flasche aus in die Flasche hineinragt, und andererseits ein Clip-Element, um einen Docht so am Dochthalter zu fixieren, dass der Docht im Gebrauch des Duftspenders teilweise in den flüssigen Duftstoff eintaucht und teilweise aus dem Duftstoff herausragt.

Bei einem erfindungsgemäßen Verfahren zur Herstellung eines erfindungsgemäßen Duftspenders wird zunächst in der erfindungsgemäßen Weise mittels eines Clip-Elements ein Docht am Dochthalter befestigt. Der Dochthalter kann dann mit dem Docht in üblicher Weise in eine Öffnung der Flasche des Duftspenders eingeführt werden, so dass der Docht nach unten in die Flasche hängt. Anschließend kann die Flasche mit dem flüssigen Duftstoff befüllt und schließlich die Öffnung der Flasche mit einer Kappe verschlossen werden.

Die abhängigen Ansprüche und die weitere Beschreibung enthalten besonders vorteilhafte Ausgestaltungen und Weiterbildungen der Erfindung, wobei der erfindungsgemäße Dochthalter und das erfindungsgemäße Verfahren auch analog zu den auf einen Duftspender gerichteten abhängigen Ansprüchen weitergebildet sein können. Ebenso können die auf den Duftspender und den Dochthalter gerichteten Ansprüche auch analog zu den abhängigen Verfahrensansprüchen weitergebildet sein.

Wie bereits eingangs beschrieben, ist es günstig, wenn der Dochthalter eine sich in eine Längsrichtung erstreckende Form aufweist. In einer besonders einfachen Ausgestaltung kann daher das Clip-Element einen sich quer zur Längsrichtung des Dochthalters erstreckenden Bügel (im Folgenden auch "Klemmbügel" genannt) aufweisen, welcher gelenkig an einer Längskante des Dochthalters befestigt ist und unter Klemmung des Dochts zur anderen Längsachse des Dochthalters hinübergeschwenkt und dort beispielsweise mittels geeigneter zusammenwirkender Rastmittel fixiert ist.

Um die Herstellungskosten für den Dochthalter möglichst gering zu halten, ist in einer besonders bevorzugten Ausführungsform der Dochthalter leiterartig mit zwei sich in Längsrichtung erstreckenden Holmen und sich quer zu den Holmen erstreckenden, die Holme verbindenden Sprossen ausgebildet. Vorzugsweise kann dabei der Klemmbügel mit einem Ende an einem der Holme gelenkig befestigt sein. Er wird dann zur Halterung des Dochts zum anderen Holm herübergeklappt und an diesem mit seinem freien Ende befestigt, wobei der Docht zwischen dem Klemmbügel einerseits und einer Sprosse des leiterförmigen Dochthalters andererseits festgeklemmt wird. Dabei kann die besagte Sprosse, welche gemeinsam mit dem verschwenkbaren Klemmbügel das eigentliche Clip-Element bildet, ebenfalls nach Art eines feststehenden Bügels ausgebildet sein. D. h. diese Sprosse erstreckt sich vorzugsweise nicht unmittelbar zwischen den beiden Holmen, sondern ist - je nach Art und Dicke des zu klemmenden Dochts - von dem beweglichen Bügel weg ein wenig aus der Leiterebene heraus bauchig ausgeformt. Zur Befestigung des freien Endes des Klemmbügels an dem zweiten Holm können am Klemmbügel und am Holm geeignete zusammenwirkende Rastmittel angeformt sein.

Ein solcher Dochthalter kann kostengünstig und daher besonders bevorzugt in einem Spritzgussverfahren einstückig aus Kunststoff hergestellt sein, wobei der bewegliche Bügel mittels eines Filmscharniers mit dem Holm verbunden ist.

An einem solchen Dochthalter wird vorzugsweise ein Docht befestigt, welcher aus einem länglichen Streifen aus Dochtmaterial, beispielsweise einem streifenförmigen Fließ, besteht, dessen Breite im Wesentlichen der Breite des Dochthalters entspricht. Dabei sollte der Docht eher noch etwas breiter als der Dochthalter sein, so dass er seitlich etwas über die seitlichen Längskanten der Dochthalters hervorragt. Bei einer bevorzugten Variante wird bei der Montage der Dochtstreifen um eine Sprosse im oberen, an der Flaschenöffnung befindlichen Bereich des leiterartigen Dochthalters so herumgelegt und nach unten am Dochthalter entlang geführt, dass der Docht im Bereich des unterhalb dieser Sprosse angeordneten Klemmbügels doppelt liegt und von dort weiter nach unten in die Flasche hängt. Durch das Herumschlagen des Dochts um die Sprosse des Dochthalters und die gemeinsame Klemmung der jeweiligen Enden des Dochtstreifens unterhalb der Sprosse mittels des Klemmbügels ist eine besonders sichere Halterung des Dochts am Dochthalter gewährleistet, ohne dass die Bestückung des Dochthalters mit dem Docht besonders aufwändig ist.

Der Klemmbügel kann in beliebiger Form ausgestaltet sein, beispielsweise als einfacher, vorzugsweise ein wenig aus der Leiterebene herausgebogener Steg, der im geschlossenen Zustand die beiden Holme verbindet.

Es kann aber auch ein leiterartiger Bügel mit zwei sich in Längsrichtung des Dochthalters erstreckenden Holmen und sich quer zu den Holmen erstreckenden, die Holme verbindenden Sprossen verwendet werden. Dabei ist ein Holm des Bügels an einer Längskante an einem der Holme des Dochthalters gelenkig befestigt und zur Halterung des Dochts und der leiterartigen Bügel komplett zum anderen Holm herübergeklappt, wobei dann der freie Holm des Bügels mit dem anderen Holm des Dochtträgers verbunden wird. Bei dieser Konstruktion wird der Docht entlang der gesamten Länge des Dochtträgers an mehreren Stellen durch die Sprossen des Dochthalters und des leiterartigen Bügels gehalten. Diese Konstruktion bietet sich insbesondere bei automatischen Bestückungen an, bei denen ein einfacher Bestückungsautomat eingesetzt werden soll, mit dem ein Herumlegen des Dochtstreifens um eine Sprosse des Dochthalters nicht durchführbar ist.

Um die Halterung weiter zu verbessern, weist das Clip-Element auf einer zum Docht gewandten Seite besonders bevorzugt Vorsprünge, beispielsweise Domen, Stege oder dergleichen auf, die in das Dochtmaterial eindringen.

Der Dochthalter ist vorzugsweise in einem Flaschenhals der Flasche längs verschiebbar, d. h. herausziehbar, gelagert, um so - wie eingangs beschrieben - die Abdampfrate des Duftspenders einzustellen. Hierzu weist der Dochthalter bevorzugt an seinen Längskanten Vorsprünge auf, beispielsweise Rastnasen, um den Dochthalter in verschiedenen Höheneinstellungen im Flaschenhals festzuhalten. Die Flasche weist bevorzugt einen Flaschenhals mit einer Engstelle auf. Die Abmessungen des Dochts sind bevorzugt so bemessen, dass die Engstelle mit Dochtmaterial ausgefüllt ist, wenn der Docht mit Duftstoff getränkt ist. Der Docht verschließt dann im etwas aufgequollenen Zustand sicher den Flaschenhals, und die Abdampfoberfläche ist nur durch den oberhalb der Engstelle aus dem Flaschenhals herausragenden Dochtteil definiert.

Damit der Verbraucher zur Einstellung der Höhenposition des Dochthalters im Flaschenhals den Dochthalter nicht berühren muss, ist der Dochthalter obenseitig bevorzugt mit einer Kappe verbunden. Der Verbraucher kann dann einfach an der Kappe ziehen, um den Dochtanteil außerhalb des Flaschenhalses zu verlängern, oder die Kappe wieder herunterdrücken, um den Docht tiefer in die Flasche hineinzuschieben. Bei der Herstellung des Duftspenders wird daher besonders bevorzugt bereits dafür gesorgt, dass die Öffnung der Flasche derart mit der Kappe verschlossen wird, dass der Dochthalter unlösbar mit der Kappe gekoppelt wird. Hierzu gibt es verschiedenste Möglichkeiten:

Eine Möglichkeit wird z. B. in der bereits zitierten FR 2, 683 149 beschrieben. Bei dieser Ausführung sind oben am Dochthalter Rastelemente angeordnet, welche mit innerhalb der Kappe angeordneten passenden Rastelementen so zusammenwirken, dass beim Aufschrauben der Kappe auf die Flasche, in der sich der Dochthalter befindet, die Rastmittel ineinander eingreifen und somit eine Sicherung des Dochthalters an der Kappe erfolgt.

Eine andere interessante Lösung zeigt die ebenfalls bereits zitierte GB 738, 173. Hier weist der Dochthalter an seinem oberen Ende eine Scheibe auf, deren Außendurchmesser so gewählt ist, dass sie sich beim Aufschrauben der Kappe auf den Flaschenhals gerade durch das Innengewinde der Kappe hindurchpressen lässt und dann im Gewinde innerhalb der Kappe gehalten wird. Diese Konstruktion hat den Vorteil, dass keine zusätzlichen Rastelemente innerhalb des Deckels notwendig sind, welche den Materialaufwand für den Deckel erhöhen würden. Jedoch hat die Verwendung einer solchen Scheibe am oberen Ende des Dochthalters den Nachteil, dass die Scheibe vor dem Aufschrauben der Kappe die Öffnung der Flasche ganz abdeckt und daher eine Befüllung des Dochthalters mit dem Duftstoff nur vor dem Einschieben des Dochthalters in die Flasche möglich ist.

Daher weist bei einem besonders bevorzugten Ausführungsbeispiel der Dochthalter obenseitig einen Haltering auf, dessen Außendurchmesser so an den Durchmesser eines Innengewindes der Kappe angepasst ist, dass der Dochthalter relativ zur Kappe drehbar in der Kappe gelagert wird. Die Verwendung eines solchen Rings hat den Vorteil, dass der Dochthalter mit dem Docht in die leere Flasche eingeschoben werden kann und anschließend durch den Ring hindurch eine Befüllung der Flasche mit dem Duftstoff möglich ist. Dennoch kann vorteilhaft - wie dies zuvor beschrieben wird - das Innengewinde in der Kappe zur Halterung des Dochthalters an der Kappe genutzt werden.

Ein weiterer Nachteil der in der GB 738,173 beschriebenen Konstruktion besteht darin, dass die Scheibe am oberen Ende des Dochthalters beim Aufschrauben der Kappe durch das Gewinde gedrückt und in der geklemmten Stellung gehalten wird. Wenn der Verbraucher an der Kappe zieht, um den Docht nach oben zu verstellen, und dabei der Docht im Flaschenhals etwas festgeklemmt ist, d. h. der Zug an der Haltescheibe etwas stärker wird, kann die Verbindung zwischen Kappe und Dochthalter gelöst werden. Da aber andererseits die Kappe - sowohl aus optischen Gründen als auch zur Vermeidung eines Verlusts der Kappe - auf dem Dochthalter befestigt sein sollte, muss der Verbraucher die Kappe wieder auf den Dochthalter aufstecken. Dies ist zwar möglich, indem der Verbraucher einfach die Kappe wieder auf den Dochthalter aufdrückt und die Flasche durch Aufschrauben der Kappe dicht verschließt, so dass sich die Scheibe wieder durch das Gewinde bis oben in die Kappe hindurch hineindrückt. Jedoch neigen viele Verbraucher dazu, zu versuchen, die Kappe wieder auf den Dochthalter aufzusetzen, während der Dochthalter in der aktuellen Position gehalten wird. Hierzu müssen sie den Dochthalter mit dem getränkten Docht mit dem Finger berühren, was als unangenehm empfunden wird. Zudem ermüdet die Verbindung zwischen Haltescheibe und Gewinde beim mehrfachen Lösen der Kappe vom Dochthalter, so dass schließlich die Kappe relativ leicht vom Dochthalter gelöst werden kann.

Um dies zu vermeiden, weist die Kappe bei einem bevorzugten Ausführungsbeispiel der Erfindung in einem axialen Abschnitt zwischen dem Innengewinde und der Innenfläche der Deckeloberseite, d. h. oberhalb des Innengewindes, einen Freischnitt auf, in welchem der Haltering frei drehbar gelagert ist. Der Haltering, welcher aufgrund seiner Ringform im Gegensatz zu einer Scheibe flexibler zusammenpressbar ist, kann sich bei einer solchen Konstruktion wieder in radialer Richtung ausdehnen und entspannen, sobald er den freigeschnittenen Bereich oberhalb des Innengewindes beim Aufschrauben der Kappe auf den Flaschenhals erreicht hat, und wird so sicher innerhalb des Freischnitts frei drehbar gehalten. Es sind dann bereits erheblich stärkere Kräfte erforderlich, um den Dochthalter mit Gewalt von der Kappe zu lösen.

Um die Fixierung des Halterings in der Kappe weiter zu erleichtern, weist der Haltering des Dochthalters an einer äußeren Umfangsfläche bevorzugt einen zum Innengewinde der Kappe passenden Gewindeabschnitt auf. Die Kappe, der Flaschenhals bzw. dessen Außengewinde und der Haltering sind dabei so ausgebildet, dass sich ein auf einer Oberkante des Flaschenhalses der Flasche aufliegender Haltering beim Aufschrauben der Kappe auf das Außengewinde des Flaschenhalses zwangsläufig im Innengewinde der Kappe bis in den Freischnitt hineinschraubt. Hierzu sind Dochthalter und Flasche außerdem so ausgebildet, dass sie nicht gegeneinander frei drehbar sind. Bei dem bereits oben beschriebenen Ausführungsbeispiel ist dies dadurch gegeben, dass der im Querschnitt rechteckige Dochthalter durch die im Querschnitt passende, rechteckige Engstelle im Flaschenhals geführt ist. Ein Entfernen des Halterings bzw. des Dochthalters aus der Kappe ist dann nur möglich, indem der Haltering, welcher ja frei drehbar innerhalb des Freischnitts gelagert ist, in eine Position gebracht wird, so dass der Gewindeabschnitt auf dem Haltering wieder in das Innengewinde der Kappe eingreift und dann wieder aus der Kappe herausgeschraubt wird. Um dies zu vermeiden, können vorzugsweise am Gewindeabschnitt des Halterings und/oder am Innengewinde der Kappe Blockiermittel angeordnet sein, die ein Zurückschrauben des Gewindeabschnitts des Halterings von dem Freischnitt aus in das Innengewinde der Kappe verhindern.

Die Erfindung wird im Folgenden unter Hinweis auf die beigefügten Figuren anhand von Ausführungsbeispielen noch einmal näher erläutert. Hieraus ergeben sich auch weitere Einzelheiten und Vorteile der Erfindung. Es zeigen:
- Figur 1: eine perspektivische Ansicht (mit Teilschnitt in einem Bereich der Flasche) eines Duftspenders gemäß einem ersten Ausführungsbeispiel im Gebrauch,
- Figur 2: eine perspektivische Ansicht eines erfindungsgemäßen Dochthalters gemäß einem ersten Ausführungsbeispiel ohne Docht
- Figuren 3 bis 5: eine perspektivische Ansicht des Dochthalters gemäß Figur 2 in verschiedenen Situationen bei der Befestigung eines Dochtstreifens am Dochthalter,
- Figur 6: einen perspektivischen Längsschnitt durch einen Dochthalter gemäß den Figuren 2 bis 5 in einer Duftspenderflasche,
- Figur 7: einen Schnitt durch die Flasche gemäß Figur 6 in Frontalansicht,
- Figur 8: einen Längsschnitt durch den Duftspender gemäß den Figuren 6 und 7 (senkrecht zur Schnittansicht in Figur 7) im Bereich der Kappe im geschlossenen Zustand,
- Figur 9: einen Längsschnitt durch den Duftspender wie in Figur 8, jedoch im offenen Zustand,
- Figuren 10 bis 12: eine perspektivische Ansicht eines Dochthalters gemäß einem zweiten Ausführungsbeispiel in verschiedenen Situationen beim Einlegen und Fixieren eines Dochts.

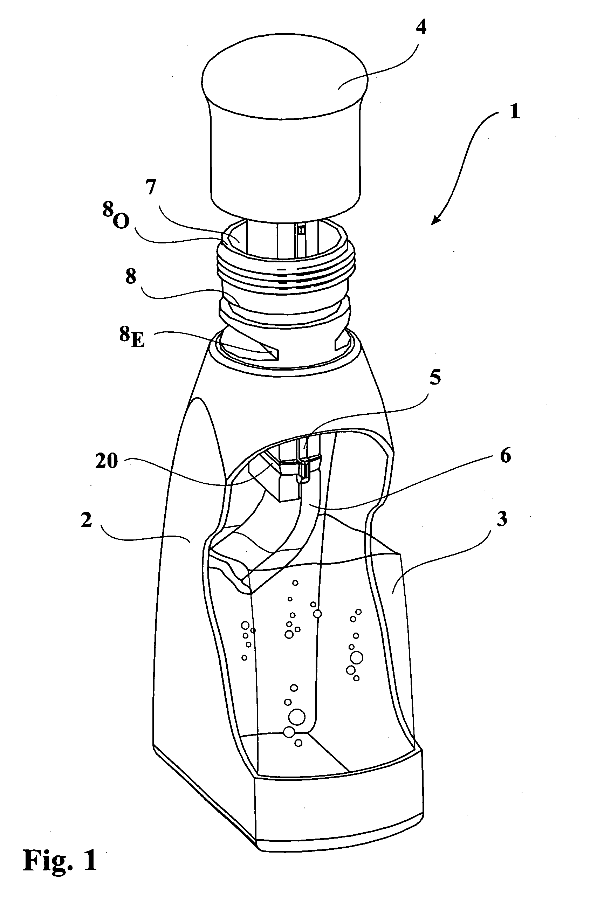

Eine erste Ausgestaltung eines erfindungsgemäßen Duftspenders 1 ist in den Figuren 1 bis 9 in verschiedensten Ansichten dargestellt. Der Duftspender 1 besteht im Wesentlichen aus einer mit flüssigem Duftstoff 3 gefüllten Flasche 2. Innerhalb der Flasche 2 ist ein Dochthalter 5, an welchem ein Docht 6 in Form eines streifenförmigen Fließmaterials angebracht ist, längs verschiebbar in einem Flaschenhals 8 der Flasche 2 angeordnet. Der Docht 6 ragt in der in Figur 1 dargestellten Gebrauchsstellung des Duftspenders mit einem oberen Ende aus dem Flaschenhals 8 heraus und ist mit seinem unteren Ende in den flüssigen Duftstoff eingetaucht. In den Figuren ist wegen der besseren Darstellbarkeit nur ein Teil des Dochts gezeigt. In der Realität ist der Docht normalerweise so lang, dass er selbst in der höchsten Stellung des Dochthalters 5 in der Flasche 2 zumindest mit einem Ende noch bis zum Flaschenboden reicht.

An seinem oberen Ende ist der Dochthalter 5 mit einer Kappe 4 unlösbar verbunden, so dass ein Verbraucher durch Hochziehen und Niederdrücken der Kappe 4 einstellen kann, wie weit der Dochthalter 5 mit dem Docht 6 aus dem Flaschenhals 8 herausragt. Durch die Einstellung des Anteils des Dochts 5, der aus dem Flaschenhals 8 herausragt, kann die Abdampfrate eingestellt werden, d. h. wie viel Duftstoff pro Zeiteinheit vom Duftspender 1 an die Umgebungsluft abgegeben wird.

Es wird an dieser Stelle darauf hingewiesen, dass die Duftspenderflasche 2 grundsätzlich jede beliebige Form aufweisen kann. Erforderlich ist lediglich, dass sie auf der Oberseite eine geeignete Öffnung aufweist, in die ein Dochthalter 5 mit einem Docht 6 hineingeschoben werden kann.

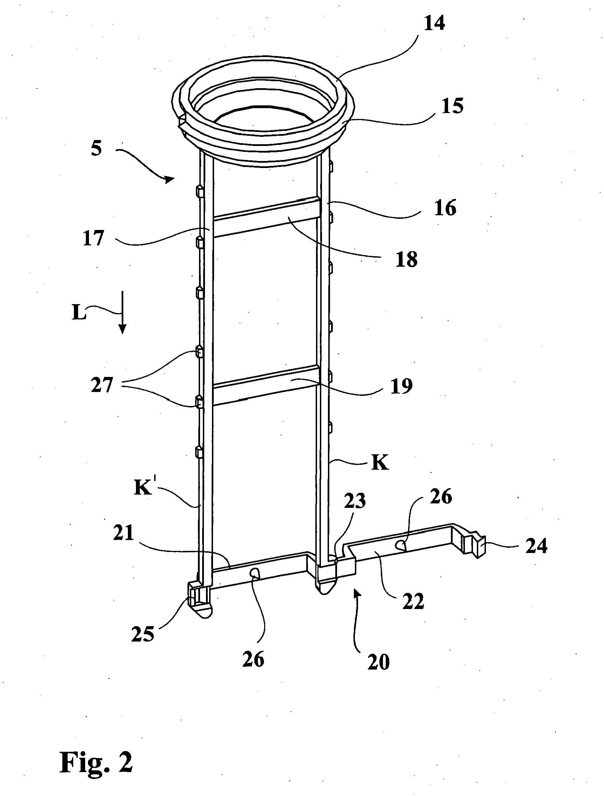

In Figur 2 ist ein besonders einfach und kostengünstig herstellbarer sowie ebenso einfach und kostengünstig mit einem Docht 6 bestückbarer Dochthalter 5 detaillierter dargestellt. Dieser Dochthalter 5 weist eine sich in einer Längsrichtung L erstreckende, längliche Form auf. Nach dem Bestücken mit dem Docht 6 wird der Dochthalter 5 in dieser Längsrichtung L in die Flasche 2 eingeschoben, wie dies in den Figuren 1 sowie 6 bis 9 dargestellt ist.

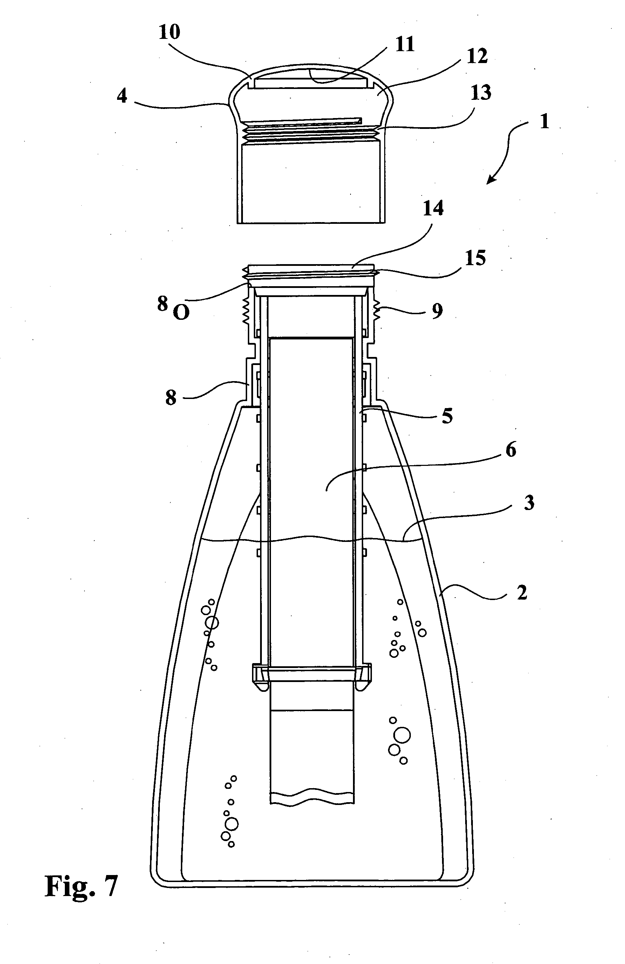

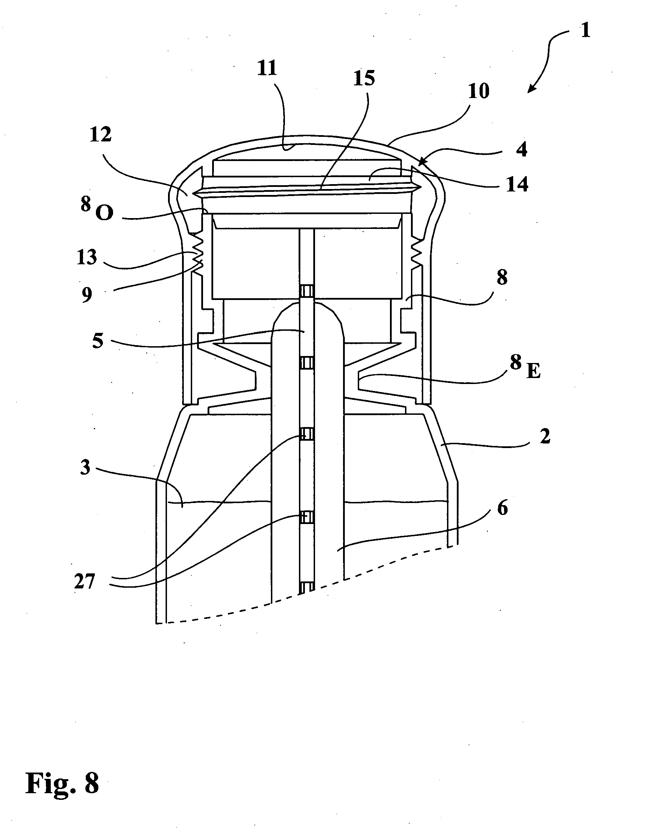

Der Dochthalter 5 ist nach Art einer Leiter aufgebaut, mit zwei Holmen 16, 17, die durch Sprossen 18, 19, 21 verbunden sind. Am oberen Ende sind die beiden Holme 16, 17 des leiterförmigen Dochthalters 5 mit einem Haltering 14 verbunden. Dieser Haltering 14 liegt bei einem geschlossenen Duftspender 1 auf der Oberkante 8_{O} des Flaschenhalses auf (siehe Figuren 7 und 8).

An dem dem Haltering 14 gegenüberliegenden, unteren Ende des leiterförmigen Dochthalters 5 sind die Holme 16, 17 durch eine Sprosse 21 miteinander verbunden, welche nach Art eines Bügels ausgebildet ist, d. h. welche von den Holmen 16, 17 weg aus der Leiterebene heraus ein wenig hervorspringt. An dieser Stelle ist außerdem über ein Filmscharnier 23 ein beweglicher Klemmbügel 22 mit dem einen Holm 16 verbunden. Sowohl die Sprosse 21 (im Folgenden auch feststehender Bügel 21 genannt) als auch der bewegliche Bügel 22 weisen auf ihrer Innenseite jeweils Domen 26 auf. Alternativ oder zusätzlich können anstelle dieser Domen auch scharfe Stege oder ähnliche Vorsprünge an den Bügeln 21, 22 angeformt sein. Der Dochthalter 5 ist einschließlich des Klemmbügels 22 einstückig aus Spritzguss-Kunststoff hergestellt.

Am freien Ende des Klemmbügels 26 ist eine Rastnase 24 angeformt. Dementsprechend befindet sich am anderen Holm 17 in der gleichen Höhe, in der am ersten Holm 16 der Bügel beweglich befestigt ist, eine passende Rastöse 25, so dass der bewegliche Klemmbügel 22, nachdem er zum anderen Holm 17 herübergeschwenkt ist, mit der Rastnase 24 in der Rastöse 25 verrastet werden kann. Der feststehende Bügel 21 und der bewegliche Bügel 22 bilden hier ein Clip-Element 20 zur Halterung eines Dochtstreifens. Dabei müssen der feststehende Bügel 21 und der bewegliche Bügel 22 nicht zwingend auf gleicher Höhe am Dochthalter 5 angeordnet sein, sondern können auch in einem (vorzugsweise aber kurzen) Abstand gegeneinander bzw. übereinander versetzt sein.

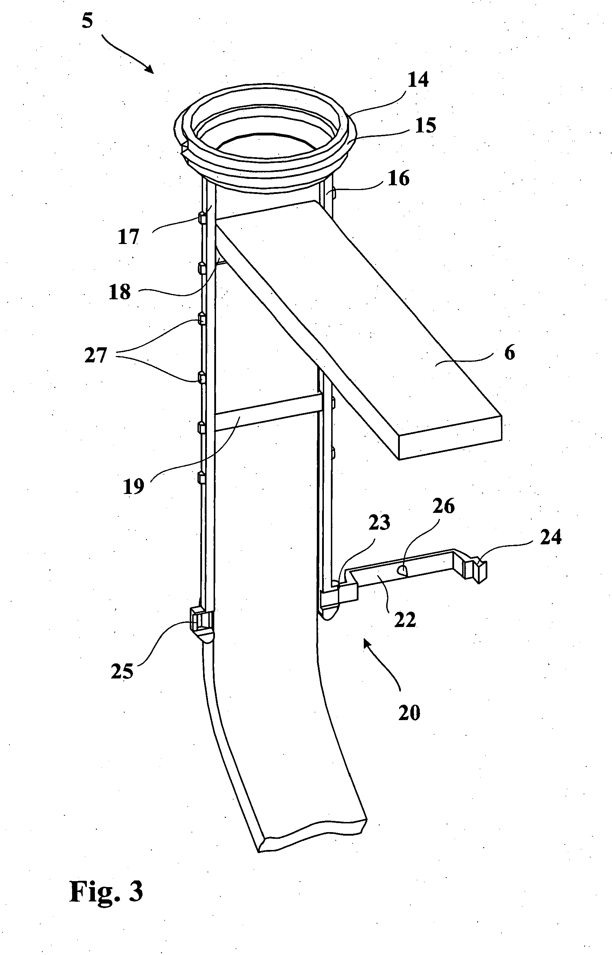

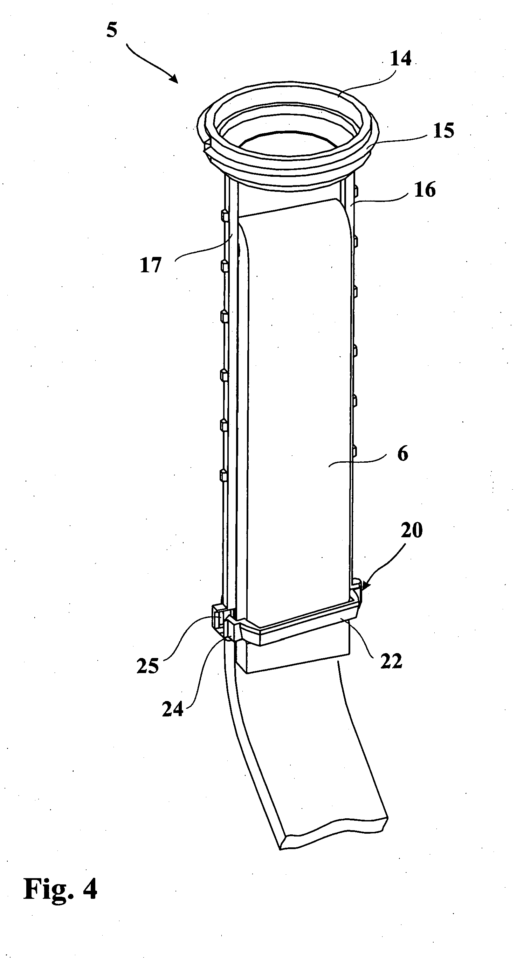

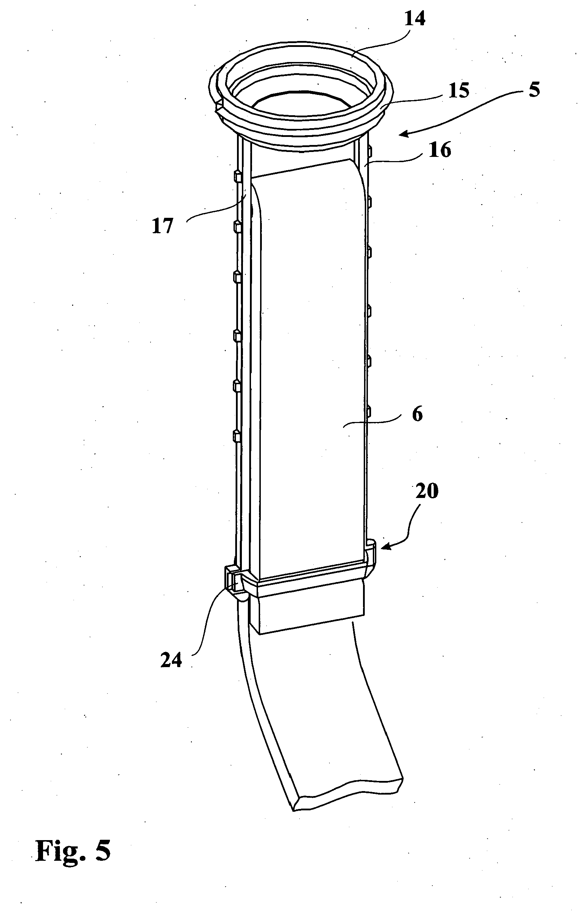

Die Figuren 3 bis 5 zeigen eine Sequenz des Arbeitsablaufs bei einem Bestücken des Dochthalters 5 gemäß Figur 2 mit einem sich in Längsrichtung L des Dochthalters 5 erstreckenden Dochtstreifen 6 aus Fließmaterial. Wie in Figur 3 dargestellt, wird der Dochtstreifen 6 einfach um die oberste, nahe dem Haltering 14 befindliche Sprosse 18 gelegt und mit beiden Enden nach unten zwischen die das Clip-Element 20 bildenden Bügel 21, 22 gelegt. Anschließend wird, wie in den Figuren 4 und 5 dargestellt, der bewegliche Klemmbügel 22 herübergeschwenkt und die am Klemmbügel 22 endseitig angeformte Rastnase 24 in die Rastöse 25 gedrückt. Damit ist der Docht 6 im Bereich des Clip-Elements 20 sicher festgeklemmt. Der gesamte Bestückungsablauf ist im Gegensatz zu den bisherigen Einfädelmethoden manuell sehr schnell und einfach durchführbar. Grundsätzlich ist eine derartige Bestückung mit einer entsprechend ausgestalteten Maschine auch automatisch möglich. Durch den neuen Dochthalter können somit die Herstellungskosten des Duftspenders erheblich reduziert werden, da insbesondere der Personalkostenanteil gesenkt werden kann.

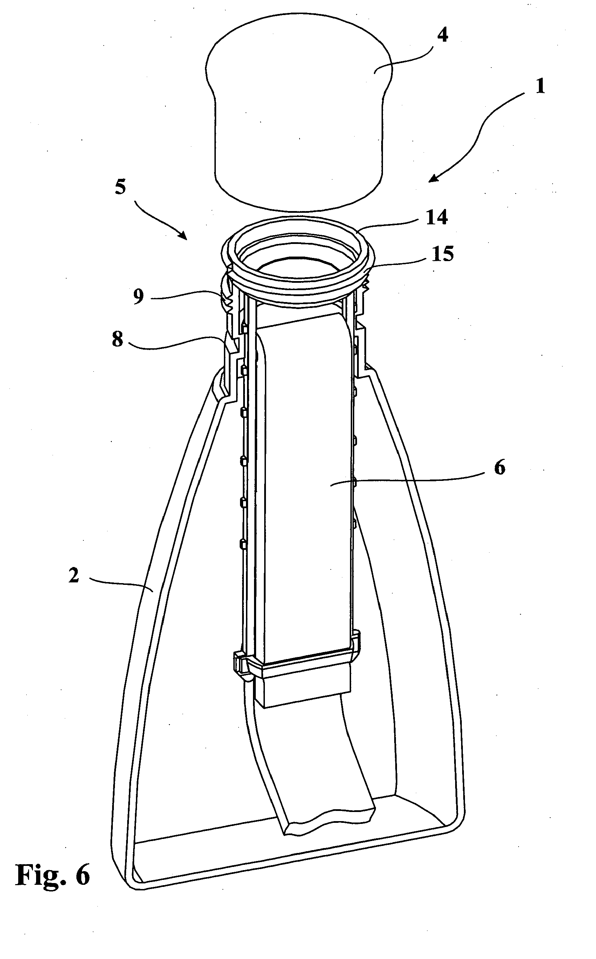

Nach dem Bestücken wird der Dochthalter 5 dann, wie in den Figuren 6 und 7 dargestellt, in eine leere Flasche 2 eingeführt, so dass der Haltering 14 auf der Oberkante 8_{O} des Flaschenhalses 8 der Flasche aufliegt.

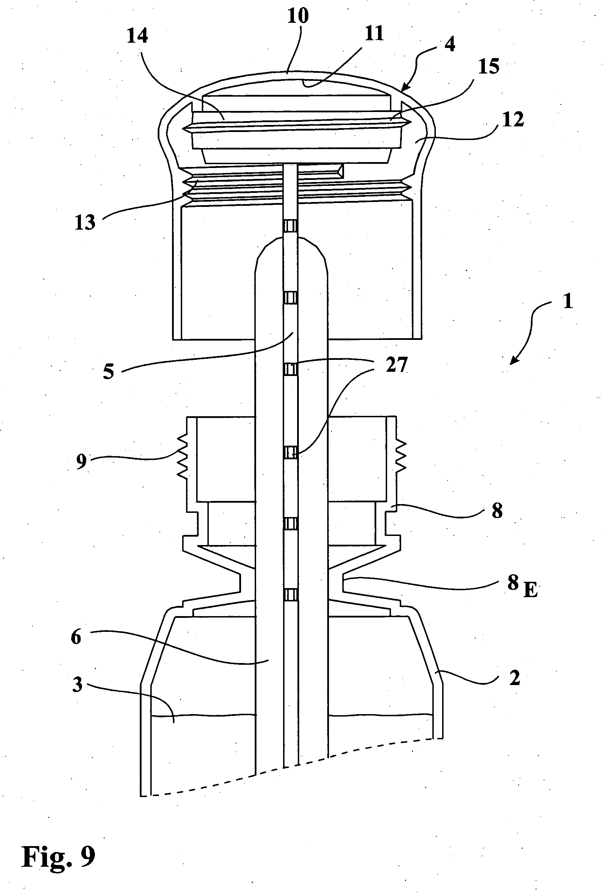

Wie in den Figuren 8 und 9, welche einen Längsschnitt durch den Flaschenhals 8 senkrecht zu dem Schnitt in Figur 7 zeigen, deutlich zu erkennen ist, weist der Flaschenhals 8 eine Engstelle 8_{E} auf. Diese Engstelle 8_{E} ist im Querschnitt im Wesentlichen rechteckig. Die Dicke des Dochts 5 ist so bemessen, dass der Docht 5, wenn er wie dargestellt zweifach übereinander liegt, die Engstelle 8_{E} ausfüllt. Hierzu entspricht die Breite des Dochtstreifens 6, anders als dies in den Figuren dargestellt ist, im Wesentlichen der Breite des Dochthalters 5 bzw. der Docht ist sogar vorzugsweise geringfügig breiter, so dass er seitlich über den Dochthalter herausragt. Lediglich um den Dochthalter 5 und die Lage des Dochts 6 am Dochthalter 5 besser darzustellen, ist der Dochtstreifen 6 in den Figuren etwas schmaler dargestellt. Wenn sich der Docht 6 dann mit dem flüssigen Duftstoff vollsaugt, quillt er noch etwas auf und schließt so die Engstelle 8_{E} sicher ab, so dass, wenn die Flasche 2 umkippt, die Flüssigkeit nicht sofort auslaufen kann. Im Übrigen hat dann auch die Oberfläche des Duftstoffs in der Flasche 2 keinen Einfluss mehr auf die Abdampfrate, sondern die Abdampfrate wird allein durch den Abschnitt des Dochts 6 bestimmt, der sich oberhalb der Engstelle 8_{E} befindet.

Um den Dochthalter 5 innerhalb des Flaschenhalses 8 auf verschiedenen Höhen zu halten, sind an den Holmen 16, 17 außenseitig Vorsprünge 27 angeordnet, welche mit einer oberhalb der Engstelle 8_{E} am Flaschenhals 8 angeordneten, ringförmigen Einengung zusammenwirken, indem die Vorsprünge jeweils unter ausreichendem Zug durch diese Einengung hindurch gezogen bzw. geschoben werden können, aber ohne äußere Kräfte auf dieser Einengung aufliegen.

Die Fixierung des Dochthalters 5 in der Kappe 4 ist ebenfalls in den Figuren 7 bis 9 dargestellt. Zunächst wird der Dochthalter, wie in Figur 7 gezeigt, vollständig in die Flasche 2 eingeschoben, bis der Haltering 14 auf der Oberkante 8_{O} des Flaschenhalses 8 aufliegt. An seinem äußeren Umfang weist der Haltering 14 einen Gewindeabschnitt 15 auf, welcher hier genau aus einem Gewindegang besteht. Der Außendurchmesser des Halterings 14 sowie das Gewinde 15 sind an den Außendurchmesser des Flaschenhalses 8 sowie an ein Außengewinde 9 am Flaschenhals 8 angepasst. Die Kappe 4 weist innen ein passendes Innengewinde 13 auf. Dieses Innengewinde der Kappe 4 läuft jedoch nicht bis an die Innenfläche 11 der Oberseite 10 der Kappe heran, sondern in dem axial oberen Bereich zwischen Innengewinde 13 und der Innenfläche 11 der Oberseite 10 der Kappe 4 befindet sich ein sich radial und axial erstreckender Freiraum, der hier als Freischnitt 12 bezeichnet wird.

Nachdem eine mit Dochthalter 5 und Docht 6 bestückte Flasche 2 durch den Haltering 14 hindurch mit Duftstoff 3 automatisch befüllt wurde, kann dann ebenfalls automatisch die Kappe 4 aufgeschraubt werden. Da der Dochthalter 5 mit dem Docht 6 im Flaschenhals 8 nicht verdreht werden kann, schraubt sich dabei der Gewindeabschnitt 15 auf dem Haltering 14 zwangsläufig durch das Innengewinde 13 der Kappe 4 bis in den Freischnitt 12 hinein. Bei fest aufgeschraubter Kappe 4 dient der Haltering 14 als Dichtung zwischen Kappe 4 und Oberkante des Flaschenhalses 8.

Wird dann vom Verbraucher die Kappe 4 von der Flasche 2 abgeschraubt, um den Duftspender 1 in Gebrauch zu nehmen, so bleibt der Haltering 14 frei drehbar im Freischnitt 12 der Kappe 4 hängen, so dass der gesamte Dochthalter 5 mit dem Deckel nach oben aus der Flasche 2 gezogen wird. Vorzugsweise sind das Innengewinde 13 und/oder der Gewindeabschnitt 15 so ausgebildet, dass ein Zurückschrauben unmöglich ist. Z. B. kann durch eine entsprechende Ausformung des Innengewindes 13 am oberen Ende, d. h. an dem Gewindeausgang zum Freischnitt 12 hin, dafür gesorgt werden, dass zwar der Gewindeabschnitt 15 beim maschinellen Aufschrauben der Kappe 4 auf die Flasche 2 leichtgängig in den Freischnitt 12 hineingeschraubt werden kann, dass es andererseits nicht möglich ist, dass der frei drehbar im Freischnitt 12 befindliche Gewindeabschnitt 15 wieder in das Innengewinde 13 hineingeführt und so wieder aus der Kappe 4 herausgeschraubt werden kann. Der Dochthalter 5 ist somit unlösbar mit der Kappe 4 verbunden und die Kappe 4 kann nicht mehr verloren gehen.

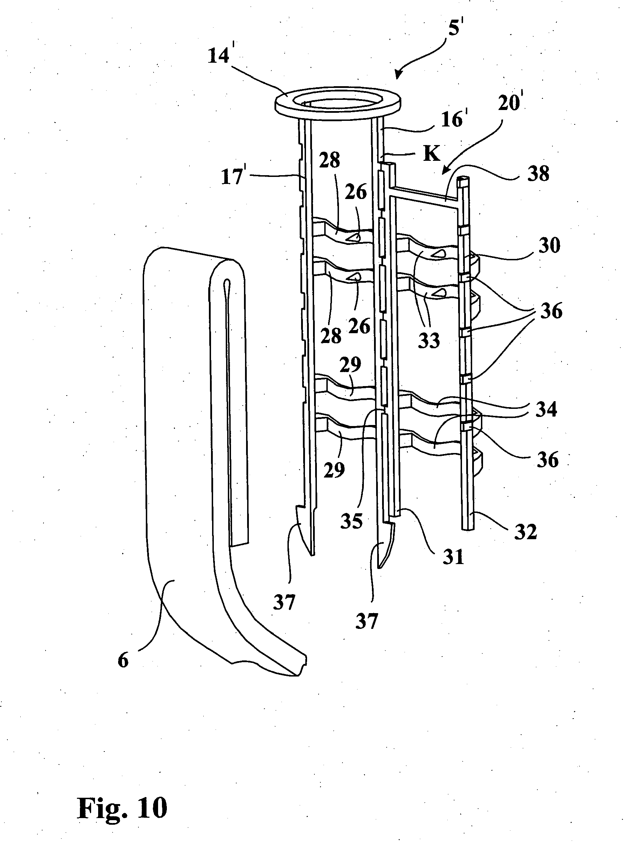

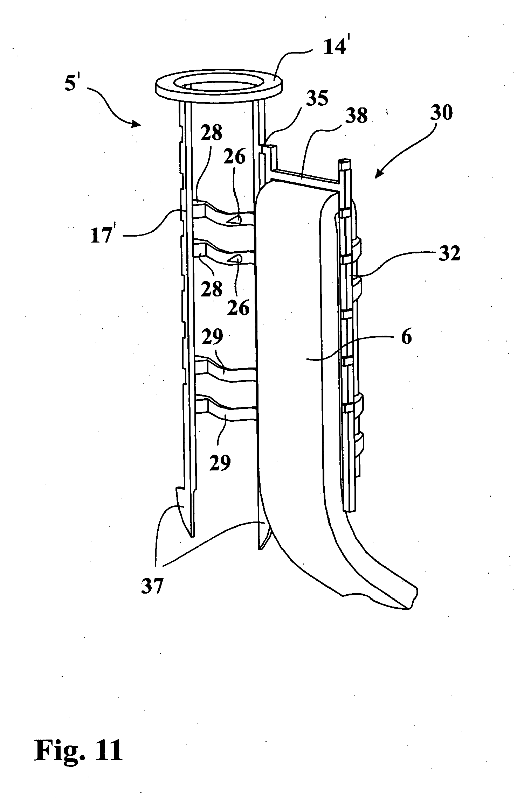

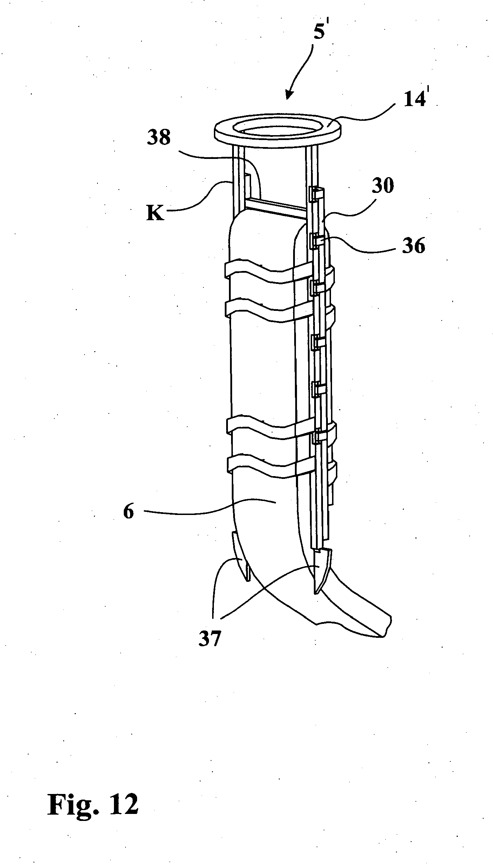

Die Figuren 10 bis 12 zeigen ein alternatives Ausführungsbeispiel eines erfindungsgemäßen Dochthalters 5'. Ein wesentlicher Unterschied zu dem in den Figuren 1 bis 9 gezeigten Dochthalter 5 besteht darin, dass hier auch der Bügel 30 leiterförmig aufgebaut ist, mit zwei sich in Längsrichtung L erstreckenden Holmen 31, 32, die hier über vier Sprossen 33, 34 miteinander verbunden sind. Dabei sind diese Sprossen 33, 34 jeweils bügelförmig ausgebildet, d. h. sie erstrecken sich in einer geschwungenen Form etwas aus der Leiterebene heraus, analog wie die Bügel 21, 22 beim ersten Ausführungsbeispiel des erfindungsgemäßen Dochthalters. Lediglich im oberen Endbereich der Holme 31, 32 ist eine gerade zwischen den Holmen 31, 32 verlaufende weitere Sprosse 38 zur Stabilisierung angeordnet.

Ähnlich wie die bügelförmigen Sprossen 33, 34 sind bei diesem Dochthalter 5' auch die Sprossen 28, 29 zwischen den Holmen 16', 17' des leiterartigen Dochthalters 5' bügelartig ausgestaltet.

Diese Holme 16', 17' sind wieder, wie beim ersten Ausführungsbeispiel, obenseitig durch einen Haltering 14' verbunden. Dabei trägt dieser Haltering 14' jedoch, anders als beim ersten Ausführungsbeispiel, kein Innengewinde, sondern ist stattdessen etwas dünner und somit flexibler ausgestaltet, so dass er beim Aufschrauben einer Kappe auf eine mit einem Dochthalter bestückte Flasche leichter nach oben durch das Innengewinde 13 bis in den Freischnitt 12 hineingedrückt wird. Hierzu ist der Durchmesser des Halterings 14' entsprechend an das Innengewinde der Kappe angepasst, so dass der Haltering 14' gerade so weit flexibel zusammengedrückt werden kann, dass er durch das Gewinde 13 passt und dann sicher innerhalb des Freischnitts 12 gehalten wird.

Der Bügel 30 ist mit einem Holm 31 an einer Längskante K über ein Filmscharnier 35 mit einem der Holme 16' des Dochthalters 5' gelenkig verbunden, so dass der Bügel 30 insgesamt zum anderen Holm 17' herübergeschwenkt werden kann und dort mit entsprechenden Rastnasen 36, welche sich am zweiten Holm 32 des Bügels 30 befinden, fixiert werden kann. Bei diesem zweiten Ausführungsbeispiel bildet folglich der komplette leiterförmige Dochthalter 5' mit sämtlichen Sprossen 28, 29 gemeinsam mit dem leiterförmig aufgebauten Bügel 30 das Clip-Element 20'. Die Rastnasen 36 dienen hier gleichzeitig zur Klemmung innerhalb des Flaschenhalses 8, d. h. zur Einstellung der Höhenposition des Dochthalters 5' in der Flasche 2.

Auch dieses zweite Ausführungsbeispiel ist kostengünstig einstückig aus Kunststoff mit einem Spritzgussverfahren herstellbar.

Anders als bei dem ersten Ausführungsbeispiel wird bei der Montage des Dochtstreifens 6 innerhalb des Dochthalters 5' der Dochtstreifen 6 hier nicht um einen Holm 18 herumgelegt. Dies hat den Vorteil, dass eine maschinelle Bestückung noch einfacher wird. Der Docht 6 wird stattdessen, wie in den Figuren 10 bis 12 dargestellt, einfach doppelt zwischen dem leiterförmigen Teil des Dochthalters 5', welches mit dem Haltering 14 verbunden ist, und dem leiterförmigen Bügel 30 eingelegt und der Bügel 30 umgeschwenkt und verrastet. Zur Sicherung des Dochts 6 innerhalb des Dochthalters 5' weist zumindest ein Teil der Sprossen 28, 33 innenseitig Domen 26 oder dergleichen auf, die in das Dochtmaterial eindringen. Ein Nachteil dieses Dochthalters 5' besteht jedoch darin, dass ein flauschigeres Dochtmaterial (bzw. ein Docht 6, welcher stärker aufquillt, sobald er mit dem Duftstoff getränkt ist) benutzt werden muss, um sicherzustellen, dass der Docht 6 die Engstelle innerhalb des Flaschenhalses 8 vollständig ausfüllt.

An den unteren - d. h. dem Haltering 14' gegenüberliegenden - Enden des leiterförmigen Dochthalters 5' befinden sich an den Holmen 16', 17' nach außen hervorstehende, hakenartige Ausformungen 37, die dafür sorgen, dass der Dochthalter 5' nicht mehr ohne weiteres wieder ganz aus der Flasche 2 herausgezogen werden kann. Bei dem ersten Ausführungsbeispiel kann eine solche Rückhaltesicherung bereits durch das Filmscharnier 23 und die Rastöse 25 bzw. die Rastnase 24 des Clip-Elements 20 realisiert werden, wobei aber selbstverständlich auch hier zusätzliche hakenartige Ausformungen an den Enden der Holme 16, 17 angeordnet sein können.

Es wird abschließend noch einmal darauf hingewiesen, dass es sich bei den in den Figuren dargestellten Duftspendern und Dochthaltern nur um Ausführungsbeispiele handelt, welche vom Fachmann in vielfacher Hinsicht variiert werden können, ohne den Rahmen der Erfindung zu verlassen. So sind insbesondere auch die verschiedensten Kombinationen der gezeigten Dochthalter möglich, d. h. dass beispielsweise ein Haltering wie bei dem zweiten Ausführungsbeispiel an einem Dochthalter gemäß dem ersten Ausführungsbeispiel verwendet wird und umgekehrt. Es wird außerdem der Vollständigkeit halber darauf hingewiesen, dass die Verwendung der unbestimmten Artikel "ein" bzw. "eine" nicht ausschließt, dass die betreffenden Merkmale auch mehrfach vorhanden sein können.

### Bezugszeichenliste

- 1: Duftspender
- 2: Flasche
- 3: Duftstoff
- 4: Kappe
- 5,5': Dochthalter
- 6: Docht
- 7: Flaschenöffnung
- 8: Flaschenhals
- 8_{O}: Oberkante des Flaschenhalses
- 8_{E}: Engstelle
- 9: Außengewinde
- 10: Deckeloberseite
- 11: Innenfläche der Deckeloberseite
- 12: Freischnitt
- 13: Innengewinde
- 14, 14': Haltering
- 15: Gewindeabschnitt
- 16, 16': Holm
- 17, 17': Holm
- 18: Sprosse
- 19: Sprosse
- 20, 20': Clip-Element
- 21: Sprosse
- 22: Bügel
- 23: Filmscharnier
- 24: Rastnase
- 25: Rastöse
- 26: Dorn
- 27: Vorsprung
- 28: Sprosse
- 29: Sprosse
- 30: Bügel
- 31: Holm
- 32: Holm
- 33: Sprosse
- 34: Sprosse
- 35: Filmscharnier
- 36: Rastnase
- 37: Ausformung
- 38: Sprosse
- L: Längsrichtung
- K, K': Längskante

## Patentansprüche

1. Duftspender (1) zur Abgabe eines flüssigen, flüchtigen Duftstoffes (3) in die Umgebungsluft,
- mit einer den flüssigen Duftstoff (3) aufnehmenden Flasche (2) mit einer durch eine Kappe (4) verschließbaren Öffnung (7),
- und mit einem von der Öffnung (7) aus in die Flasche(2) hineinragenden Dochthalter (5, 5') mit einem daran befestigten Docht (6), welcher im Gebrauch des Duftspenders (1) teilweise in den flüssigen Duftstoff (3) eintaucht und teilweise aus dem Duftstoff (3) herausragt,
**dadurch gekennzeichnet,**
**dass** der Docht (6) mittels eines Clip-Elements (20, 20') am Dochthalter fixiert ist.

2. Duftspender nach Anspruch 1, **dadurch gekennzeichnet, dass** der Dochthalter (5, 5') eine sich in eine Längsrichtung (L) erstreckende Form aufweist und dass das Clip-Element (20, 20') einen sich quer zur Längsrichtung (L) des Dochthalters (5, 5') erstreckenden Bügel (22, 30) umfasst, welcher gelenkig an einer Längskante (K) des Dochthalters (5, 5') befestigt ist und unter Klemmung des Dochtes (6) zur anderen Längskante (K') des Dochthalters (5, 5') herübergeschwenkt und dort fixiert ist.

3. Duftspender nach Anspruch 2, **dadurch gekennzeichnet, dass** der Dochthalter (5, 5') leiterartig mit zwei Holmen (16, 17, 16', 17') und sich quer zu den Holmen (16, 17, 16', 17') erstreckenden, die Holme (16, 17, 16', 17') verbindenden Sprossen (18, 19, 21, 28, 29) ausgebildet ist, und dass der Bügel (22, 30) mit einem Ende an einem der Holme (16, 16') gelenkig befestigt ist und zur Halterung des Dochts (6) zum anderen Holm (17, 17') herübergeklappt und an diesem mit einem anderen Ende befestigt ist, wobei der Docht (6) zwischen dem Bügel (22, 30) einerseits und einer Sprosse (21, 28, 29) des Dochthalters (5, 5') andererseits festgeklemmt ist.

4. Duftspender nach Anspruch 3, **dadurch gekennzeichnet, dass** der Bügel (30) ebenfalls leiterartig mit zwei Holmen (31, 32) und sich quer zu den Holmen (31, 32) erstreckenden, die Holme (31, 32) verbindenden Sprossen (33, 34, 38) ausgebildet ist, und dass ein Holm (31) des Bügels (30) an einer Längskante (K) an einem der Holme (16') des Dochthalters (5') gelenkig befestigt ist und zur Halterung des Dochts (6) zum anderen Holm (17') herübergeklappt ist, wobei der andere Holm (32) des Bügels (30) mit dem anderen Holm (17') des Dochtträgers (5') verbunden ist.

5. Duftspender nach einem der Ansprüche 1 bis 6, **dadurch gekennzeichnet, dass** der Docht (6) aus einem länglichen Streifen aus Dochtmaterial besteht, dessen Breite im Wesentlichen der Breite des Dochthalters (5, 5') entspricht.

6. Duftspender nach einem der Ansprüche 3 bis 5, **dadurch gekennzeichnet, dass** der Docht (6) um eine Sprosse (18), die in einem oberen, an der Flaschenöffnung (7) befindlichen Bereich des leiterartigen Dochthalters (5) angeordnet ist, so herumgelegt ist und nach unten in die Flasche (2) hängt, dass der Docht (5) im Bereich eines unterhalb dieser Sprosse (18) angeordneten Bügels (22) doppelt liegt.

7. Duftspender nach einem der Ansprüche 1 bis 6, **dadurch gekennzeichnet, dass** das Clip-Element (20, 20') auf einer zum Docht (6) gewandten Seite in das Dochtmaterial eindringende Vorsprünge (26) aufweist.

8. Duftspender nach einem der Ansprüche 1 bis 7, **dadurch gekennzeichnet, dass** die Flasche (2) einen Flaschenhals (8) mit einer Engstelle (8_{E}) aufweist und die Abmessungen des Dochts (6) so bemessen sind, dass die Engstelle (8_{E}) mit Dochtmaterial ausgefüllt ist, wenn der Docht (6) mit Duftstoff getränkt ist.

9. Duftspender nach einem der Ansprüche 1 bis 8, **dadurch gekennzeichnet, dass** der Dochthalter (5, 5') in einem Flaschenhals (8) der Flasche (2) längs verschiebbar gelagert ist.

10. Duftspender nach Anspruch 9, **dadurch gekennzeichnet, dass** der Dochthalter (5) an seinen Längskanten (K, K') Vorsprünge (27) aufweist.

11. Duftspender nach einem der Ansprüche 1 bis 10, **dadurch gekennzeichnet, dass** der Dochthalter (5, 5') mit der Kappe (4) verbunden ist.

12. Duftspender nach Anspruch 11, **dadurch gekennzeichnet, dass** die Kappe (4) eine Schraubkappe (4) mit einem Innengewinde (13) ist und dass der Dochthalter (5, 5') obenseitig einen Haltering (14, 14') aufweist, dessen Außendurchmesser so an den Durchmesser des Innengewindes (13) der Kappe (4) angepasst ist, dass der Dochthalter (5, 5') relativ zur Kappe (4) drehbar in der Kappe (4) gehalten wird.

13. Duftspender nach Anspruch 12, **dadurch gekennzeichnet, dass** die Kappe (4) in einem axialen Abschnitt zwischen dem Innengewinde (13) und der Innenfläche (11) der Deckeloberseite (10) einen Freischnitt (12) aufweist, in welchem der Haltering (14) frei drehbar gelagert ist.

14. Duftspender nach Anspruch 12 oder 13, **dadurch gekennzeichnet, dass** der Haltering (14) des Dochthalters (5) an einer äußerem Umfangsfläche einen zum Innengewinde (13) der Kappe (4) passenden Gewindeabschnitt (15) aufweist, und die Kappe (4), der Flaschenhals (8) und der Haltering (14) so ausgebildet sind, dass sich ein auf einer Oberkante (8_{O}) des Flaschenhalses (8) der Flasche (2) aufliegender Haltering (14) bei einem Aufschrauben der Kappe (4) auf ein Außengewinde (9) des Flaschenhalses (8) im Innengewinde (13) der Kappe (4) bis in den Freischnitt (12) hineinschraubt.

15. Duftspender nach Anspruch 14, **dadurch gekennzeichnet, dass** das Innengewinde (13) der Kappe (4) und/oder der Gewindeabschnitt (15) am Haltering (14) des Dochthalters (5) so ausgebildet sind, dass ein Zurückschrauben des Halterings (14) aus dem Freischnitt (12) der Kappe (4) verhindert wird.

16. Dochthalter (5, 5') für einen Duftspender (1) nach einem der Ansprüche 1 bis 15,
mit zumindest einem Halterungselement (14, 14'), um den Dochthalter (5, 5') in einer den flüssigen Duftstoff (3) aufnehmenden Flasche (2) des Duftspenders (1) so zu halten, dass er von einer Öffnung (7) der Flasche (2) aus in die Flasche (2) hineinragt,
und mit einem Clip-Element (20, 20'), um einen Docht (6) so am Dochthalter (5, 5') zu fixieren, dass der Docht (6) im Gebrauch des Duftspenders (1) teilweise in den flüssigen Duftstoff (3) eintaucht und teilweise aus dem Duftstoff (3) herausragt.

17. Verfahren zur Herstellung eines Duftspenders (1) nach einem der der Ansprüche 1 bis 15, mit folgenden Verfahrensschritten,
- Fixieren eines Dochts (6) an einem Dochthalter (5, 5') mittels eines Clip-Elements (20, 20'),
- Einführen des Dochthalters (5, 5') mit dem Docht (6) in eine Öffnung (7) einer Flasche (2) des Duftspenders (1), so dass der Docht (6) nach unten in die Flasche (2) hängt,
- Befüllen der Flasche (2) mit einem flüssigen Duftstoff (3),
- Verschließen der Öffnung (7) der Flasche (2) mit einer Kappe (4).

18. Verfahren nach Anspruch 17, **dadurch gekennzeichnet, dass** der Dochthalter (5) leiterartig mit zwei Holmen (16, 17) und sich quer zu den Holmen (16, 17) erstreckenden, die Holme (16, 17) verbindenden Sprossen (18, 19, 21) ausgebildet ist, und dass der Docht (6) zunächst um eine Sprosse (18), die in einem oberen, sich nach der Einführung des Dochthalters (5) in die Flasche (2) nahe der Flaschenöffnung (7) befindlichen Bereich des leiterartigen Dochthalters (5) angeordnet ist, herumgelegt wird und mittels eines Bügels (22), der unterhalb dieser Sprosse (18) mit einem Ende an einem der Holme (16) gelenkig befestigt ist, fixiert wird, indem der Bügel (22) zum anderen Holm (17) herübergeklappt und an diesem befestigt wird, wobei der Docht (6) zwischen dem Bügel (22) einerseits und einer Sprosse (21) des Dochthalters (5) andererseits festgeklemmt wird.

19. Verfahren nach Anspruch 18 oder 19, **dadurch gekennzeichnet, dass** die Öffnung (7) der Flasche (2) so mit der Kappe (4) verschlossen wird, dass der Dochthalter (5) unlösbar mit der Kappe (4) verbunden wird.
